# EUROPÄISCHE PATENTANMELDUNG

(11) **EP 1 025 815 A1**
(43) Veröffentlichungstag der Anmeldung: **09.08.2000**
(21) Anmeldenummer: 00810005.9
(22) Anmeldetag: 05.01.2000
(51) Int. Cl.: A61F 2/34

(54) **Künstliche Gelenkschale**

(30) Priorität: 04.02.1999 EP 99810096
(71) Anmelder: Sulzer Orthopedics Ltd., 6340 Baar (CH)
(72) Erfinder: Schürch, Markus, 8957 Spreitenbach (CH); Roberts, Paul, Tutshill, Chepstow, NP67BX (GB); Grigoris, Peter, Dr.med., Kelvinbridge, Glasgow G128DN (GB)
(74) Vertreter: Sulzer Management AG

(57) **Zusammenfassung**

Eine künstliche Gelenkschale (1) für die Implantation in eine entsprechende Kavität im Knochen eines Patienten weist eine konvexe Aussenfläche auf, welche bei der Implantation in der Kavität zu liegen kommt, sowie eine konkave Lagerfläche zur Aufnahme einer Gelenkkugel. Die Dicke (d) der künstlichen Gelenkschale (1) liegt im Bereich von etwa 2.5 mm bis etwa 3 mm.

## Beschreibung

Die Erfindung betrifft eine künstliche Gelenkschale gemäss dem Oberbegriff des unabhängigen Patentanspruchs.

Beim Auftreten von Schäden an einer Gelenkpfanne, z.B. am acetabulum, eines Patienten ist es bekannt, künstliche Gelenkpfannen zu implantieren. Solche künstlichen Gelenkpfannen sind in vielen Ausführungsvarianten bekannt. Sie umfassen jeweils eine metallische Schale (oder etwas Ähnliches wie z.B. eine auf eine nichtmetallische Schalle aufgetragene metallische Schicht oder ein metallisches Geflecht) sowie einen in der Schale angeordneten Einsatz (Inlay), der üblicherweise aus Metall, Keramik oder Kunststoff (z.B. Polyethylen) hergestellt ist. Deratige künstliche Gelenkpfannen werden seit geraumer Zeit implantiert - sie sind sehr stabil, aufgrund ihrer Konstruktion (Schale plus Einsatz) und der erforderlichen Dicke der jeweiligen Materialien sind sie jedoch vergleichsweise voluminös.

Dies hat zur Folge, dass selbst bei relativ kleinen Beschädigungen der natürlichen Gelenkpfanne - z.B. des acetabulums - eines Patienten, speziell bei Beschädigungen des Knorpels bei intaktem Knochen, bei der Vorbereitung des Knochens (bzw. der natürlichen Gelenkpfanne) für die Implantation einer künstlichen Gelenkpfanne relativ viel Knochen entfernt werden muss. Die Vorbereitung des Knochens für die Implantation, also das Entfernen von Knochen, erfolgt typischerweise mit Hilfe eines hierfür vorgesehenen Knochenfräsers. Insbesondere bei jungen Patienten bedeutet dies, dass bei der Vorbereitung des Knochens für die Aufnahme der künstlichen Gelenkpfanne schon zu einem sehr frühen Zeitpunkt relativ viel Knochensubstanz verloren geht, sodass für eine später vielleicht einmal erforderliche Reoperation dann nicht mehr so viel Knochensubstanz zur Verfügung steht. Vor allen Dingen dann, wenn der Schaden an der Gelenkpfanne klein ist, aber dennoch eine künstliche Gelenkpfanne implantiert werden muss, ist die Implantation der konventionellen künstlichen Hüftgelenkpfannen zwar eine gute und verlässliche Lösung, aber sie lässt auch noch einigen Raum für Verbesserungen.

Es ist daher eine Aufgabe der Erfindung, eine künstliche Gelenkschale vorzuschlagen, für welche bei der Vorbereitung des Knochens des Patienten für die Implantation möglichst wenig Knochen entfernt werden muss, aber trotzdem eine zuverlässige Gelenkverbindung entsteht und insbesondere bei Beschädigungen des Knorpels bei intaktem Knochen eine anatomische Nachbildung des Knorpels durch die Gelenkschale erreicht wird.

Erfindungsgemäss wird die Aufgabe durch eine künstliche Gelenkschale gelöst, wie sie im unabhängigen Patentanspruch charakterisiert ist. Besonders vorteilhafte Ausführungsvarianten ergeben sich aus den abhängigen Patentansprüchen.

Die erfindungsgemässe künstliche Gelenkschale für die Implantation in eine entsprechende Kavität im Knochen eines Patienten weist eine konvexe Aussenfläche auf, welche bei der Implantation in der Kavität zu liegen kommt, sowie eine konkave Lagerfläche zur Aufnahme einer Gelenkkugel. Die Dicke der künstlichen Gelenkschale liegt im Bereich von etwa 2.5 mm bis etwa 3 mm. Mit Hilfe einer derart dünnwandigen Schale ist es möglich, bei geringen Beschädigungen der natürlichen Gelenkpfanne so viel Knochen wie möglich zu erhalten und gleichzeitig eine zuverlässige Gelenkverbindung zu erhalten. Insbesondere bei Beschädigungen des Knorpels bei intaktem Knochen wird eine anatomische Nachbildung des Knochens durch die Gelenkschale erreicht.

Vorzugsweise ist die Gelenkschale im wesentlichen aus Metall hergestellt, wobei insbesondere Kobalt-Chrom-Legierungen in Frage kommen, aber auch andere Materialien. Die Gelenkfläche (also die Innenfläche der Gelenkschale) kann beschichtet sein, hierfür kommen ggf. auch andere Materialien in Frage wie z.B. verschleissarme Kunststoffe. Auch die Aussenfläche oder zumindest Teile davon - hierauf wird weiter unten noch genauer eingegangen - kann beschichtet sein, z.B. mit Titan.

Bei einem weiteren vorteilhaften Ausführungsbeispiel weist die Aussenfläche im Bereich des Poles einen nach aussen abstehenden zapfenartigen Vorsprung auf, der von einer ringförmigen Abflachung umgeben ist. Die Höhe des Zapfens ist so bemessen, dass die Stirnfläche des Zapfens gegenüber der fiktiven Fortsetzung der konvexen Aussenfläche zurückgesetzt ist. Dieses Ausführungsbeispiel hat herstellungstechnische Vorteile, aber auch Vorteile, die mit der Implantation der Gelenkschale zusammenhängen.

Bei der Herstellung der Gelenkschale wird nämlich zunächst die Aussenfläche hergestellt, die hier eine aufwendigere Geometrie aufweist als die Innenfläche. Ist die Aussenfläche fertiggestellt, muss die Innenfläche - das ist die Gelenkfläche oder Gleitfläche des Gelenks - hergestellt werden. Dies ist bei der zu erzielenden Dicke der Gelenkschale nicht einfach, da der auf der Aussenfläche bereits bearbeitete Rohling nicht so ohne weiteres in das Futter z.B. einer Drehmaschine oder in den Werkzeughalter einer Schleif- oder Poliermaschine eingespannt werden kann, weil sonst eine Deformation erfolgen kann. Bei der Genauigkeit, mit welcher die Gelenkfläche hergestellt werden muss, darf es nicht zu Deformationen kommen. Hierzu dient der im Bereich des Poles vorgesehene, nach aussen abstehende Zapfen. Dieser kann auf einfache Weise von einem Futter einer Drehmaschine aufgenommen werden und auf diese Weise kann dann die Innenfläche durch Drehen erstellt werden, ohne dass es dabei zu Deformationen kommt, weil die Gelenkschale eben im Bereich des Poles gehalten werden kann.

Weil die Höhe des Zapfens so bemessen ist, dass die Stirnfläche des Zapfens gegenüber der fiktiven Fortsetzung der konvexen Aussenfläche zurückgesetzt ist, stösst der Zapfen beim Implantieren der Gelenkschale in die konkave Kavität, die üblicherweise mit einem Formfräser vorbereitet wird, nicht gegen den Boden der Kavität, sondern lässt hier einen geringen Zwischenraum.

Vorzugsweise ist der zapfenartige Vorsprung aus einem Material hergestellt, auf welchem Knochen nicht bzw. nur äusserst schlecht anwachsen kann. Dies ist insofern vorteilhaft, als bei einer möglicherweise zu einem späteren Zeitpunkt erforderlichen Reoperation ein Entfernen der Gelenkschale aus der Kavität dann besonders schwer wird, wenn auf die Gelenkschale im Bereich des Poles Knochen aufgewachsen ist. Besteht der Zapfen z.B. aus einer weiter oben bereits erwähnten Kobalt-Chrom-Legierung, so kann im Bereich des Poles kein Knochen auf die Aussenfläche der Gelenkschale aufwachsen.

Ein weiteres vorteilhaftes Ausführungsbeispiel der erfindungsgemässen Gelenkschale zeichnet sich dadurch aus, dass im Bereich des Äquators auf der Aussenfläche sich in Umfangsrichtung erstreckende Finnen (oder Widerhaken) vorgesehen sind, die in Richtung vom Pol weg zum Äquator hin geneigt und in Umfangsrichtung unterbrochen sind.

Diese Finnen dienen der Primärverankerung in der Kavität - also der Verankerung unmittelbar nach dem Einbringen der Gelenkschale. Bei der zementfreien Implantation muss die Gelenkschale ja eine Primärverankerung im Knochen aufweisen, zumindest so lange, bis der Knochen genügend gut auf die übrige Gelenkschale aufgewachsen ist, sodass dann eine gute Sekundärverankerung der Gelenkschale gegeben ist. Diese gute Primärverankerung wird durch die Finnen erreicht. Die Kavität zur Aufnahme der Gelenkschale wird dabei mit einem Formfräser hergestellt, dessen Durchmesser kleiner ist als der Aussendurchmesser der Gelenkschale, sodass beim Implantieren der Gelenkschale ein Pressitz der Gelenkschale im Knochen erzielt wird. Die Neigung der Finnen in Richtung vom Pol weg zum Äquator hin ermöglicht es, dass die Finnen beim Hineindrücken der Gelenkschale in die Kavität gut einbringbar ist, aber anschliessend nicht mehr ohne weitere Massnahmen entnehmbar ist. Insbesondere dienen die Finnen auch dem Schutz der eingebrachten Gelenkschale gegen eine Verkippung der Schale.

Weil die in Umfangsrichtung sich erstreckenden Finnen unterbrochen sind und die Gelenkschale einen kleineren Aussendurchmesser aufweist als die mit Hilfe des Formfräsers erstellte Kavität, wird der spongiöse Knochen aufgrund seiner elastischen Eigenschaften in die Zwischenräume (Unterbrechungen) der Finnen zurückfedern, wodurch die Gelenkschale gegen Verdrehen gesichert wird.

In einer vorteilhaften Weiterbildung der Erfindung sind mehrere Finnen in verschiedenen zur äquatorialen Ebene parallelen Ebenen vorgesehen. Die sich in Umfangsrichtung erstreckenden Finnen sind in den verschiedenen Ebenen jeweils derart unterbrochen, dass zwischen den Finnen Nuten gebildet werden, welche sich in Richtung vom Äquator zum Pol hin erstrecken bzw. in umgekehrter Richtung. Dadurch wird die bereits erläuterte Primärverankerung der Gelenkschale (Sicherung gegen Verkippen und Verdrehen) noch erhöht.

Bei einem besonders vorteilhaften Ausführungsbeispiel der Erfindung ist die Aussenfläche im Bereich zwischen den im äquatorialen Bereich angeordneten Finnen und der im Polbereich vorgesehenen ringförmigen Abflachung mit einem Material beschichtet, insbesondere mit Titan, auf welchem Knochen gut anwachsen kann. Diese Fläche macht einen vergleichsweise grossen Anteil der Aussenfläche der Gelenkschale aus und bewirkt eine gute Sekundärverankerung der Gelenkschale, sobald Knochen auf diese vorzugsweise mit Titan beschichtete Aussenfläche aufgewachsen ist. Titan ist für seine Eigenschaft bekannt, dass ein gutes Anwachsen des Knochens auf einer mit Titan beschichteten Fläche einer Prothese möglich ist und dass es sehr gut körperverträglich ist.

Besonders vorteilhaft ist es, wenn zum Beschichten der Gelenkschale ein Vakuum-Plasma-Beschichtungsverfahren eingesetzt wird, weil dabei die zu beschichtende Gelenkschale nicht unnötig stark erwärmt wird, damit die Dauerfestigkeit der Metallpfanne nicht beeinträchtigt wird und es ausserdem nicht zu Spannungen oder Verformungen kommt.

Wie bereits erläutert, kann mit einer Gelenkschale, wie sie vorstehend beschrieben ist, ein Verfahren zum Ersetzen einer natürlichen Gelenkfläche durch eine Gelenkschale mit einer künstlichen Gelenkfläche durchgeführt werden. Bei diesem Verfahren wird der Knochen mit der natürlichen Gelenkfläche mit einem Formfräser für die Aufnahme der Gelenkschale vorbereitet und anschliessend wird die Gelenkschale mit der künstlichen Gelenkfläche in den entsprechend vorbereiteten Knochen implantiert. Durch die Verwendung einer vorstehend beschriebenen Gelenkschale muss der Knochen mit Hilfe des Formfräsers nur so weit (bzw. so wenig) abgetragen werden, bis er eine Gelenkschale gemäss einem der vorangehenden Ansprüche vom Knochen aufnehmen kann. Dann wird die Gelenkschale implantiert. Auf diese Weise kann bei geringfügigen Beschädigungen der natürlichen Gelenkpfanne eines Patienten so viel Knochensubstanz wie möglich erhalten werden. Dies ist insbesondere bei jungen Patienten vorteilhaft, wo möglicherweise zu einem späteren Zeitpunkt eine Reoperation erforderlich werden kann. Für eine solche Reoperation steht dann noch ausreichen Knochenmaterial zur Verfügung.

Wie bereits vorstehend erläutert, wird der Knochen vorteilhafterweise derart vorbereitet, dass der Durchmesser der mit Hilfe des Formfräsers erzeugten Kavität zur Aufnahme der Gelenkschale im Knochen um ein vorgegebenes Mass kleiner ist als der Aussendurchmesser der Gelenkschale. Dieses Mass liegt insbesondere im Bereich von etwa 1.5 mm bis etwa 3 mm, sodass beim Implantieren der Gelenkschale ein Pressitz der Gelenkschale im Knochen erreicht wird. Dies ermöglicht eine gute Primärverankerung der Gelenkschale, sodass sehr bald nach der Operation der Patient das Gelenk wieder belasten kann.

Üblicherweise wird bei der Implantation einer solchen Gelenkschale in eine Kavität - z.B. in das acetabulum - der entsprechende Gelenkkopf des Gelenks - z.B. der Femurkopf - nicht reseziert, sondern es wird auf dem Gelenkkopf eine künstliche Gelenkkape auf dem Femurkopf angebracht als Gleitpartner für die Gelenkschale. Eine Resektion des Gelenkkopfs kann daher unterbleiben.

Im folgenden wird die Erfindung anhand der Zeichnung näher erläutert. Es zeigen, teilweise schematisch und/oder im Schnitt:
- Fig. 1: ein Ausführungsbeispiel einer erfindungsgemässen Gelenkschale in perspektivischer Ansicht
- Fig. 2: das Ausführungsbeispiel der Gelenkschale gemäss Fig. 1 in einer Aufsicht,
- Fig. 3: das Ausführungsbeispiel der Gelenkschale gemäss Fig. 1 in einem Schnitt gemäss der Linie III - III der Fig. 2,
- Fig. 4: einen Ausschnitt aus einem Schnitt durch die Wand der Gelenkschale gemäss der Linie IV - IV der Fig. 2
und
- Fig. 5: den Ausschnitt V aus Fig. 4, vergrössert.

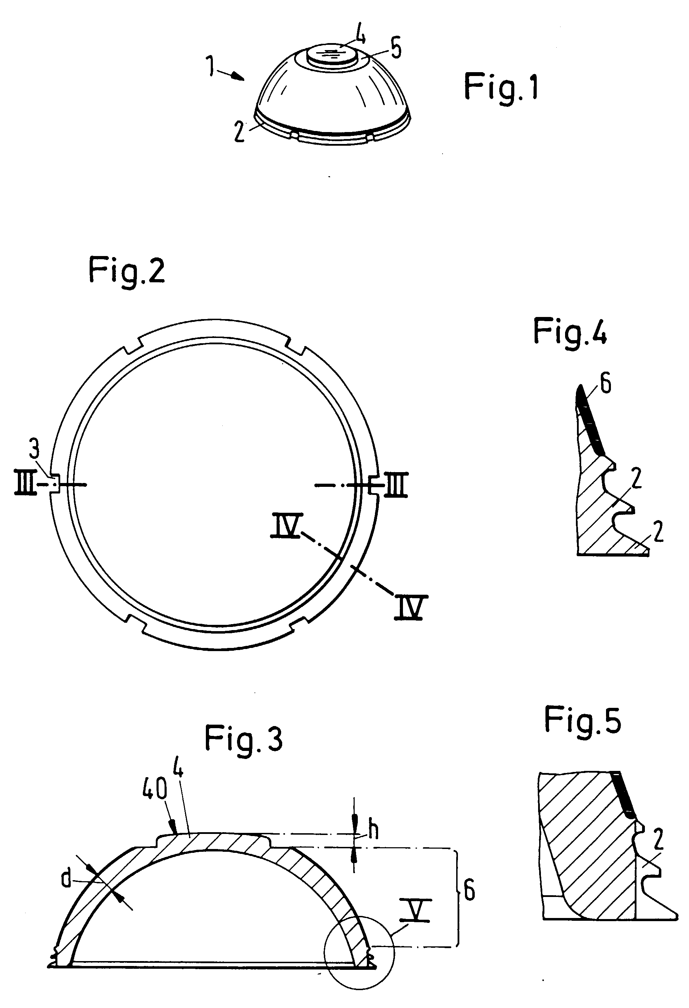

Fig. 1 zeigt in einer perspektivischen Ansicht ein Ausführungsbeispiel einer erfindungsgemässen Gelenkschale 1. Die Gelenkschale 1 weist im Bereich des Äquators in Umfangsrichtung sich erstreckende Finnen 2 auf. Diese Finnen 2 sind in Umfangsrichtung unterbrochen (siehe Fig. 2) und sind in Richtung vom Pol weg zum Äquator hin geneigt (siehe Fig. 4, Fig. 5). Diese Finnen 2 dienen einer guten zementfreien Primärverankerung der Gelenkschale 1 in einer im Knochen des Patienten vorbereiteten Kavität (nicht dargestellt). Die Finnen 2 können beim Einbringen der Gelenkschale 1 in die vorbereitete Kavität hineingleiten, verhaken sich aber dann im Knochengewebe und die Gelenkschale 1 kann nicht mehr aus der Kavität herausgleiten noch kann sie verkippen.

In Fig. 1 und in Fig. 2 erkennt man, dass in mehreren zur Ebene des Äquators parallelen Ebenen die einzelnen Finnen 2 in Umfangsrichtung unterbrochen sind, sodass zwischen den Finnen 2 Nuten 3 gebildet werden, die sich in Richtung vom Äquator zum Pol hin erstrecken bzw. in umgekehrter Richtung. Nach dem Einbringen der Gelenkschale 1 in die vorbereitete Kavität gelangt der elastische spongiöse Knochen in die Nuten 3 zwischen den Finnen 2 hinein, wodurch eine Sicherung der Gelenkschale 1 gegen Verdrehen bewirkt wird.

Die Dicke d einer solchen Gelenkschale 1 (siehe Fig. 3) liegt im Bereich von etwa 2.5 mm bis etwa 3 mm. Derartig dünnwandige Schalen ermöglichen es, den Knochen der Patienten so weit wie möglich zu erhalten, was insbesondere für junge Patienten, bei denen keine grosse Beschädigung des natürlichen Gelenks vorliegt, vorteilhaft ist, speziell im Hinblick darauf, dass später möglicherweise eine Reoperation erfolgen muss und dann immer noch genügend Knochen zur Verfügung steht für die Verankerung einer Gelenkpfanne der herkömmlichen Art.

Die Gelenkschale 1 ist vorzugsweise vollständig metallisch, kann aber auch auf der Innenwand eine Beschichtung mit einem extrem abriebfesten Kunststoff aufweisen. Materialien, welche für die Herstellung einer solchen Gelenkschale 1 bevorzugt verwendet werden können, sind Chrom-Kobalt Legierungen (z.B. die Chrom-Kobalt Legierung, die unter dem Namen PROTASUL 21-WF bei der Anmelderin erhältlich ist), welche sehr gute mechanische und tribologische Eigenschaften aufweisen. Aber auch andere metallische Materialien (z.B. Titan, Stahl) kommen grundsätzlich in Frage. Alternativ kann die Schale auch mehr oder weniger vollständig aus Kunstoff hergestellt sein (z.B. aus Polyethylen), wobei die Kunststoffschale auf der Aussenwand mit einem knochenkompatiblen Material beschichtet sein kann, damit der Knochen besser auf die Schale aufwachsen kann.

Im Bereich des Poles weist die Aussenfläche der Gelenkschale 1 einen nach aussen abstehenden zapfenartigen Vorsprung 4 auf. Dieser Zapfen 4 ist von einer ringförmigen Abflachung 5 umgeben. Die Höhe h dieses Zapfens 4 ist so bemessen, dass die Stirnfläche 40 des Zapfens 4 gegenüber der fiktiven Fortsetzung der Aussenfläche der Gelenkschale 1 zurückgesetzt ist, der Zapfen 4 reicht also nicht bis an die fiktive Fortsetzung der Aussenfläche der Gelenkschale 1 heran. Dies hat den Vorteil, dass beim Implantieren der Gelenkschale 1 in die im Knochen vorbereitete Kavität der Zapfen 4 nicht gegen den Boden der Kavität anstösst und so ein weiteres Einbringen der Gelenkschale 1 in die Kavität hinein nicht behindert. Vor allen Dingen hat der Zapfen 4 aber eine besondere Bedeutung für die Herstellung der Gelenkschale 1.

Bei der Herstellung der Gelenkschale 1 wird nämlich zunächst die Aussenfläche der Gelenkschale 1 hergestellt, einschliesslich des Zapfens 4 und der Finnen 2. Ist die Aussenfläche fertiggestellt, so muss die halbfertige Gelenkschale 1 für die Herstellung der Innenfläche von einem Werkstückhalter aufgenommen werden. Dies erfolgt am Zapfen 4, der z.B. vom Futter einer Drehmaschine, vom Werkstückhalter einer Schleifmaschine oder einer Poliermaschine aufgenommen werden kann (der Durchmesser des Zapfens 4 kann z.B. in einem Bereich von etwa 10-25 mm liegen). Danach wird die Innenfläche (Gelenkfläche, Artikluationsfläche) hergestellt. Bei der Herstellung der Innenfläche darf keine wesentliche Deformation der halbfertigen Gelenkschale 1 erfolgen, weshalb die Gelenkschale 1 im Bereich ihres Poles, nämlich am Zapfen 4, festgehalten wird. Bei am Zapfen 4 festgehaltener, halbfertiger Gelenkschale 1 wird dann die Innenfläche durch Drehen, Schleifen und Polieren hergestellt. Die Art und Weise, wie man derartige Gelenkschalen und insbesondere deren Innenflächen herstellen kann, ist beispielsweise in der WO-A-97/38650 beschrieben.

Die Aussenfläche der Gelenkschale 1 weist - wie bereits beschrieben - im Bereich des Äquators die Finnen 2 auf und im Bereich des Poles den ebenfalls bereits beschriebenen Zapfen 4. Da nun die Gelenkschale 1 vorzugsweise aus einer Chrom-Kobalt Legierung hergestellt sein kann, andererseits eine Chrom-Kobalt Legierung kein gutes Anwachsen des Knochens auf der Aussenfläche der Gelenkschale 1 ermöglicht (keine gute Sekundärverankerung), ist es vorzugsweise so, dass die Gelenkschale 1 im Bereich 6 zwischen den im äquatorialen Bereich angeordneten Finnen 2 und der im Polbereich vorgesehenen Abflachung 5 mit einem Material beschichtet ist, insbesondere mit hochreinem Titan, auf welchem Knochen gut anwachsen kann. Sowohl im Bereich der Finnen 2 (also im äquatorialen Bereich) als auch im Bereich des Zapfens 4 (also im Polbereich) ist die Gelenkschale 1 auf ihrer Aussenfläche nicht beschichtet. Dort soll die Primärverkankerung der Gelenkschale 1 (also die Verankerung in der Zeit unmittelbar anschliessend an die Implantation) erfolgen.

Die Primärverankerung soll aber nicht dauerhaft die einzige Verankerung der Gelenkschale 1 bleiben. Abgesehen davon ist es auch sehr schwierig, im Bereich der Finnen 2 eine gleichmässige Beschichtung der Gelenkschale zu erreichen, und die Beschichtung könnte in diesem Bereich (scharfe Kanten) relativ leicht Schaden erleiden. Nach erfolgtem Anwachsen des Knochens auf den mit Titan beschichteten Bereich 6 wird die Verankerung der Gelenkschale 1 dort bewirkt (Sekundärverankerung). Vor allem im Hinblick darauf, dass eine Gelenkschale 1 möglicherweise zu einem späteren Zeitpunkt einmal ersetzt werden muss, ist eine Verankerung einer derartigen Gelenkschale 1 im Bereich des Poles nicht wünschenswert, weil dann beim Entfernen der Gelenkschale im Bereich des Poles (wenn dort Knochen auf die Gelenkschale aufgewachsen wäre) relativ viel Knochenmaterial verloren gehen würde und gerade im Polbereich der natürlichen Gelenkpfanne (z.B. am acetabulum) ohnehin nicht gerade sehr viel Knochenmaterial zur Verankerung einer künstlichen Gelenkpfanne zur Verfügung steht.

Die Beschichtung der Aussenfläche im Bereich 6 zwischen den Finnen 2 und dem Zapfen 4 ist besonders gut in Fig. 3, Fig. 4 und Fig. 5 zu erkennen. Sie reicht jeweils bis knapp an die Finnen 2 heran, sodass insgesamt ein grosser Bereich der Aussenfläche mit Titan (mit einer Porosität von z.B. 20 - 40%) beschichtet ist (Schichtdicke z.B. im Bereich von etwa 150 bis etwa 500 µm) und Knochen gut aufwachsen kann, sodass eine gute Sekundärverankerung der Gelenkschale 1 erreicht wird. Die Rauheit (Rauhtiefe R_{z}) kann dabei z.B. im Bereich von etwa 100 bis etwa 250 µm liegen.

Die Beschichtung des Bereichs 6 mit hochreinem Titan erfolgt vorzugsweise nach der Herstellung der Innenfläche der Gelenkschale 1. Da es zu diesem Zeitpunkt zu keinen Verformungen der Innenfläche mehr kommen darf, wird das Titan vorzugsweise mit Hilfe eines Vakuum-Plasma-Beschichtungsverfahrens in Form von Pulver aufgebracht. Bei der Beschichtung des Bereichs 6 der Gelenkschale1 mit Hilfe eines solchen Beschichtungsverfahrens bleibt die Temperatur der Gelenkschale 1 niedrig, sodass keine thermisch bedingten Deformationen auftreten können. Alternativ zu einem Vakuum-Plasma-Beschichtungsverfahren könnte eine Beschichtung des Bereichs 6 auch mittels Aufsintern von spongiosaähnlich strukturiertem Titan erfolgen.

Beim Ersetzen einer natürlichen Gelenkfläche durch eine Gelenkschale 1 mit einer künstlichen Gelenkfläche muss der Orthopäde zunächst den Knochen (z.B. das acetabulum) mit Hilfe eines Formfräsers für die Aufnahme der Gelenkschale 1 vorbereiten. Der Knochen muss dabei aber nur so weit abgetragen werden, bis er die vergleichsweise sehr dünne Gelenkschale 1 aufnehmen kann. Sodann wird die Gelenkschale 1 in die vorbereitete Kavität eingebracht.

Bei der Implantation einer solchen künstlichen Gelenkschale 1 wird normalerweise auch die natürliche Femurgelenkkugel mit einer künstlichen Femurkappe (Gelenkkappe) versehen. Zu diesem Zweck muss aber die natürliche Femurgelenkkugel nicht etwa reseziert werden, wie dies bei der Implantation von Schaftprothesen der Fall ist, sondern sie kann erhalten bleiben und wird lediglich mit einer entsprechenden Femurkappe versehen. Grundsätzlich kommen bei der Gelenkflächenpaarung zwischen der Femurkappe und der Gelenkschale sämtliche in diesem Gebiet gängigen Materialpaarungen in Frage.

Vorzugsweise ist der Durchmesser des Formfräsers zur Herstellung der Kavität im Knochen, in welchem die Gelenkschale 1 aufgenommen werden soll, um etwa 1.5 mm bis etwa 3 mm geringer als der Aussendurchmesser der Gelenkschale 1. Dadurch wird ein sicherer Pressitz der Gelenkschale 1 im Knochen erreicht.

## Patentansprüche

1. Künstliche Gelenkschale (1) für die Implantation in eine entsprechende Kavität im Knochen eines Patienten, mit einer konvexen Aussenfläche, welche bei der Implantation in der Kavität zu liegen kommt, sowie mit einer konkaven Lagerfläche zur Aufnahme einer Gelenkkugel, dadurch gekennzeichnet, dass die Dicke (d) der künstlichen Gelenkschale (1) im Bereich von etwa 2.5 mm bis etwa 3 mm liegt.

2. Künstliche Gelenkschale nach Anspruch 1, dadurch gekennzeichnet, dass die Gelenkschale (1) im wesentlichen aus Metall hergestellt ist.

3. Künstliche Gelenkschale nach Anspruch 1 oder 2, dadurch gekennzeichnet, dass die Aussenfläche im Bereich des Poles einen nach aussen abstehenden zapfenartigen Vorsprung (4) aufweist, der von einer ringförmigen Abflachung (5) umgeben ist, wobei die Höhe (h) des Zapfens (4) so bemessen ist, dass die Stirnfläche (40) des Zapfens (4) gegenüber der fiktiven Fortsetzung der konvexen Aussenfläche zurückgesetzt ist.

4. Künstliche Gelenkschale nach Anspruch 3, dadurch gekennzeichnet, dass der zapfenartige Vorsprung (4) aus einem Material hergestellt ist, auf welchem Knochen nicht bzw. nur äusserst schlecht anwachsen kann.

5. Künstliche Gelenkschale nach einem der vorangehenden Ansprüche, dadurch gekennzeichnet, dass im Bereich des Äquators auf der Aussenfläche sich in Umfangsrichtung erstreckende Finnen (2) vorgesehen sind, die in Richtung vom Pol weg zum Äquator hin geneigt und in Umfangsrichtung unterbrochen sind.

6. Künstliche Gelenkschale nach Anspruch 5, dadurch gekennzeichnet, dass mehrere Finnen (2) in verschiedenen zur äquatorialen Ebene parallelen Ebenen vorgesehen sind, wobei die in Umfangsrichtung sich erstreckenden Finnen (2) in den verschiedenen Ebenen jeweils derart unterbrochen sind, dass zwischen den Finnen Nuten (3) gebildet werden, welche sich in Richtung vom Äquator zum Pol hin erstrecken bzw. in umgekehrter Richtung.

7. Künstliche Gelenkschale nach einem der vorangehenden Ansprüche, dadurch gekennzeichnet, dass die Aussenfläche im Bereich (6) zwischen den im äqatorialen Bereich angeordneten Finnen (2) und der im Polbereich vorgesehenen ringförmigen Abflachung (5) mit einem Material beschichtet ist, insbesondere mit Titan, auf welchem Knochen gut anwachsen kann.

8. Künstliche Gelenkschale nach Anspruch 7, dadurch gekennzeichnet, dass zum Beschichten der Gelenkschale (1) ein Vakuum-Plasma-Beschichtungsverfahren eingesetzt wird.

9. Verfahren zum Ersetzen einer natürlichen Gelenkfläche durch eine Gelenkschale (1) mit einer künstlichen Gelenkfläche, bei welchem Verfahren der Knochen mit der natürlichen Gelenkfläche mit einem Formfräser für die Aufnahme der Gelenkschale (1) vorbereitet und anschliessend die Gelenkschale (1) mit der künstlichen Gelenkfläche in den entsprechend vorbereiteten Knochen implantiert wird, dadurch gekennzeichnet, dass der Knochen mit Hilfe des Formfräsers nur so weit abgetragen wird, bis er eine Gelenkschale (1) gemäss einem der vorangehenden Ansprüche vom Knochen aufnehmen kann und dass dann eine Gelenkschale (1) nach einem der vorangehenden Ansprüche implantiert wird.

10. Verfahren nach Anspurch 9, dadurch gekennzeichnet, dass der Knochen derart vorbereitet wird, dass der Durchmesser der mit Hilfe des Formfräsers erzeugten Kavität zur Aufnahme der Gelenkschale (1) im Knochen um ein vorgegebenes Mass kleiner ist als der Aussendurchmesser der Gelenkschale (1), insbesondere um ein Mass im Bereich von etwa 1.5 mm bis etwa 3 mm, sodass beim Implantieren der Gelenkschale (1) ein Pressitz der Gelenkschale (1) im Knochen erreicht wird.
